# EUROPEAN PATENT SPECIFICATION

(11) **EP 4 209 586 B1**
(45) Date of publication and mention of the grant of the patent: **29.10.2025**
(21) Application number: 21864679.2
(22) Date of filing: 02.09.2021
(51) Int. Cl.: C12N 5/0783, C12N 15/86, C07K 14/55, C07K 16/28, C07K 14/71, A61K 35/17, A61K 39/00, A61P 35/00, C07K 14/705

(54) **EXTRACELLULAR VESICLE EXPRESSING CYTOKINE AND ANTIBODY, METHOD FOR PRODUCING SAME, AND USE THEREOF**
EXTRAZELLULÄRE VESIKEL ZUR EXPRESSION VON CYTOKIN UND ANTIKÖRPER, VERFAHREN ZUR HERSTELLUNG DAVON UND VERWENDUNG DAVON
VÉSICULE EXTRACELLULAIRE EXPRIMANT UNE CYTOKINE ET UN ANTICORPS, SON PROCÉDÉ DE PRODUCTION ET SON UTILISATION

(30) Priority: 04.09.2020 KR 20200112773; 30.08.2021 KR 20210114652
(43) Date of publication of application: 12.07.2023
(73) Proprietor: Daegu Gyeongbuk Institute Of Science and Technology, Daegu 42988 (KR); Kyungpook National University Industry-Academic Cooperation Foundation, Daegu 41566 (KR)
(72) Inventor: BAEK, Moon-Chang, Daegu 42116 (KR); YEA, Kyung Moo, Daegu 43016 (KR); CHO, Hanchae, Daegu 42988 (KR)
(74) Representative: Roos, Peter
(86) International application number: PCT/KR2021/011853
(87) International publication number: WO 2022/050720

(56) References cited:
- WO-A1-2019/027847
- WO-A1-2020/172328
- WO-A2-99/16426
- JP-A- 2018 515 092
- KR-A- 20180 078 173
- WEINSTEIN-MAROM HADAS ET AL: "Membrane-attached Cytokines Expressed by mRNA Electroporation Act as Potent T-Cell Adjuvants", JOURNAL OF IMMUNOTHERAPY, vol. 39, no. 2, 1 March 2016 (2016-03-01), US, pages 60 - 70, XP093217512, ISSN: 1524-9557, DOI: 10.1097/CJI.0000000000000109
- NICOLAS BLANCHARD ET AL: "TCR activation of human T cells induces the production of exosomes bearing the TCR/CD3/zeta complex.", THE JOURNAL OF IMMUNOLOGY, vol. 168, no. 7, 1 April 2002 (2002-04-01), pages 3235 - 3241, XP055057390, ISSN: 0022-1767, DOI: 10.4049/jimmunol.168.7.3235
- WOJTA-STREMAYR DANIELA ET AL: "CD8+ T Cell Fate and Function Influenced by Antigen-Specific Virus-Like Nanoparticles Co-Expressing Membrane Tethered IL-2", vol. 10, no. 5, 6 May 2015 (2015-05-06), pages e0126034, XP055983184, Retrieved from the Internet <URL:https://journals.plos.org/plosone/article/file?id=10.1371/journal.pone.0126034&type=printable> DOI: 10.1371/journal.pone.0126034
- SHI XIAOJING, CHENG QINQIN, HOU TIANLING, HAN MENGLU, SMBATYAN GOAR, LANG JULIE E., EPSTEIN ALAN L., LENZ HEINZ-JOSEF, ZHANG YONG: "Genetically Engineered Cell-Derived Nanoparticles for Targeted Breast Cancer Immunotherapy", MOLECULAR THERAPY, ELSEVIER INC., US, vol. 28, no. 2, 1 February 2020 (2020-02-01), US , pages 536 - 547, XP055907186, ISSN: 1525-0016, DOI: 10.1016/j.ymthe.2019.11.020
- YUNSHAN YANG ; FANGMING XIU ; ZHIJIAN CAI ; JIANLI WANG ; QINGQING WANG ; YANGXIN FU ; XUETAO CAO: "Increased induction of antitumor response by exosomes derived from interleukin-2 gene-modified tumor cells", JOURNAL OF CANCER RESEARCH AND CLINICAL ONCOLOGY, SPRINGER, BERLIN, DE, vol. 133, no. 6, 12 January 2007 (2007-01-12), Berlin, DE , pages 389 - 399, XP019517726, ISSN: 1432-1335, DOI: 10.1007/s00432-006-0184-7

## Description

### Technical Field

The present disclosure relates to an extracellular vesicle expressing cytokines and antibodies, a method for producing the same, and a use thereof.

### Background Art

An extracellular vesicle (EV) refers to a nano-sized vesicle derived from cells, classified into exosomes, microvesicles, ectosomes, microparticles, membrane vesicles, nanovesicles, outer membrane vesicles, and the like depending on the secretion form and size. The extracellular vesicle is the main means of communication among cells, including nucleic acids and proteins which are the main components of cells.

Recently, it has been reported that the secretion of extracellular vesicles increases in cancer patients, and changes are observed in certain proteins that are specifically shown in the cancer patients, such that it is noticeable that the extracellular vesicles are likely to be applied as a marker for diagnosing cancer. In addition, since the extracellular vesicle is highly stable in vivo without inducing an immune response, encapsulation of drugs therein may enable selective delivery of drugs to target cells in the body, and since it is easy to separate and purify the extracellular vesicle from various cells, researches are being conducted on using extracellular vesicles as a drug delivery.

On the other hand, cancer is a disease caused by abnormal cell growth, with reports that the mortality rate of cancer patients is high that it ranks first as the cause of death in Korea. There are dozens of cancer types, mainly classified on the basis of the tissue of origin. Cancer is divided into benign tumors and malignant tumors, wherein benign tumors have relatively slow growth rate and hardly show metastasis from the primary site of the tumor to other tissues, while malignant tumors are life-threatening due to rapid growth by infiltrating into other tissues away from the primary site.

Surgery, chemotherapy, and radiotherapy are applied for the cancer treatment methods, but it has been reported that more than 50% of cancer patients who had undergone treatment die despite prolonged research. The reason why it is difficult to treat cancer despite of application of various treatment methods is that even if the cancer is surgically removed to cure the cancer patient, cancer recurs due to the metastasis of the cancer cells to other tissues of the patient, or the prognosis of cancer deteriorates drastically due to cancer cells showing resistance to anticancer drugs during or after the treatment, although the size of the tumor decreases due to anticancer drugs in the initial treatment stage.

In particular, anticancer drugs, which are commonly used chemotherapy, show anticancer effects mainly through the mechanism of inhibiting the proliferation of cancer cells, and about 60 different types have been developed. However, since most anticancer drugs nonselectively act on normal cells as well as cancer cells, causing serious side effects such as hair loss, extreme fatigue, vomiting, nausea, discoloration of the skin and nails, and side effects in the nervous system.

In order to overcome the issues of conventional anticancer drugs, targeted anticancer drugs and anticancer therapy using immune cells have been developed as treatment methods that specifically act on cancer cells. However, these treatment methods show discrepancies in treatment efficiency depending on the characteristics of the patient, such that the problem still remains unsolved, such as the development of acute inflammatory diseases due to excessive activation of the immune system. Therefore, there is a need to develop a new treatment method that is specific to cancer cells.

### [Prior Art Document]

### [Patent Document]

Korean Patent No. 10-1820264 (issued on January 12, 2018)

### Disclosure of the Invention

### Technical Goals

An object of the present disclosure is to provide an extracellular vesicle of which cytokines and antibodies are expressed on the surface of a membrane as a novel means of cancer treatment.

Another object of the present disclosure is to provide a pharmaceutical composition for preventing or treating cancer, comprising the extracellular vesicle as an active ingredient.

Another object of the present disclosure is to provide a composition for delivering a drug or bioactive substance, comprising the extracellular vesicle in which a drug or bioactive substance exhibiting therapeutic effects on cancer is encapsulated as a novel means of cancer treatment.

### Technical Solutions

The present disclosure provides an extracellular vesicle expressing cytokines and antibodies on its surface as defined in claim 1.

In addition, the present disclosure provides a pharmaceutical composition as defined in claim 10 for use in preventing or treating cancer, comprising the extracellular vesicle as an active ingredient.

In addition, the present disclosure provides a composition as defined in claim 12 for use in delivering a drug or bioactive substance, comprising the extracellular vesicle, wherein the drug or bioactive substance is encapsulated in the extracellular vesicle.

In addition, the present disclosure provides a method as defined in claim 14 for producing the extracellular vesicle, including preparing a first vector comprising a gene encoding a cytokine, a linker domain encoding a linker, and a transmembrane domain encoding a transmembrane protein; preparing a second vector comprising a gene encoding an antibody, a linker domain encoding a linker, and a transmembrane domain encoding a transmembrane protein; transforming cells with the first vector and second vector using lentivirus; and isolating the extracellular vesicles from a culture medium in which the cells transformed with the first vector and second vector are cultured.

### Advantageous Effects

According to the present disclosure, by transforming immune cells with a first vector comprising a gene encoding a cytokine and a second vector comprising a gene encoding an antibody, it is possible to prepare immune cells that express both cytokines and antibodies on the surface of a cell membrane and prepare an extracellular vesicle expressing both cytokines and antibodies on the surface from the immune cells, wherein the extracellular vesicle may induce a desired cytokine response in cells that may be targeted with a specific antibody by combining a first vector and second vector so that specific cytokines or specific antibodies may be expressed depending on the target cell, and thus may be provided as a novel anticancer drug that is safe for the human body and exhibits superior anticancer effects.

In addition, applicable for a delivery of a drug or bioactive substance to specific cancer cells by encapsulating the drug or bioactive substance that exhibits therapeutic effects on cancer therein, the extracellular vesicle of the present disclosure may be provided as a novel means of cancer treatment.

### Brief Description of Drawings

FIG. 1 is a diagram illustrating a composition of a vector for producing an extracellular vesicle of the present disclosure and a method for producing an extracellular vesicle.
FIG. 2 shows results of isolating immune cells to be used to produce extracellular vesicles of the present disclosure from a blood sample and analyzing characteristics thereof with a flow cytometer.
FIG. 3 shows results of identifying whether cytokines and/or antibodies are expressed in immune cells that are transformed to express cytokines and/or antibodies to produce extracellular vesicles of the present disclosure.
FIG. 4 shows results of evaluating the cancer cell-specific cytotoxicity of extracellular vesicles produced according to the present disclosure.
FIG. 5 shows results of evaluating the cytotoxicity of cytokine IL-2.
FIG. 6 shows results of evaluating the cancer cell specificity on the basis of antibodies expressed on the surfaces of extracellular vesicles produced according to the present disclosure.
FIG. 7 shows a result of verifying the antibody specificity of extracellular vesicles produced according to the present disclosure.
FIG. 8 shows identification of cancer cell-specific targeting upon treatment of fluorescence-stained extracellular vesicles to non-small cell lung cancer cell (A549)-grafted mice via 'fluorescence, luminescence, and computed tomography in vivo optical imaging system' and 'confocal fluorescence microscopy'.

### Best Mode for Carrying Out the Invention

The terms used herein have been selected from currently widely used general terms as much as possible in consideration of functions herein, but these may vary depending on the intentions or precedents of those skilled in the art, the emergence of new technologies, and the like. In addition, in specific cases, there are terms arbitrarily selected by the applicant, and in this case, the meaning will be described in detail in the description of the disclosure. Therefore, the terms used herein should not be defined by simple names of terms, but based on the meaning of the term and the overall contents of the present disclosure.

Unless otherwise defined, all terms used herein, including technical or scientific terms, have the same meaning as commonly understood by those skilled in the art to which the present disclosure pertains. Terms such as those defined in commonly used dictionaries should be construed as having meanings consistent with the meaning in the context of the relevant art and are not to be construed in an ideal or overly formal meaning unless clearly defined in the present application.

The numerical range includes the numerical value defined in the above range. All maximum numerical limits given herein include all lower numerical limits as clearly stated on the lower numerical limits. All minimum numerical limits given herein include all higher numerical limits as clearly stated on the higher numerical limits. All numerical limits given herein will include all better numerical ranges within a wider numerical range as clearly stated on narrower numerical limits.

Hereinafter, the present disclosure will be described in more detail.

As the development of new treatment methods specific to cancer cells is required, the inventors of the present disclosure produced an extracellular vesicle of which cytokines activating the immune system and antibodies selectively targeting cancer cells are expressed on its surface, and identified cancer cell-specific cytotoxicity thereof, thereby providing the extracellular vesicle expressing cytokines and antibodies on the surface as a means of preventing or treating cancer and a carrier for delivering a drug or bioactive substance.

The present disclosure provides an extracellular vesicle expressing cytokines and antibodies on its surface.

The extracellular vesicle may be prepared as a membrane bound cytokine (MBC) platform which consists of a "cytokine or antibody-encoding gene-linker domain-transmembrane domain", such that cytokines or antibodies are bound to a linker linked to a transmembrane protein so as to be expressed in a form attached to the cell membrane instead of being secreted extracellularly.

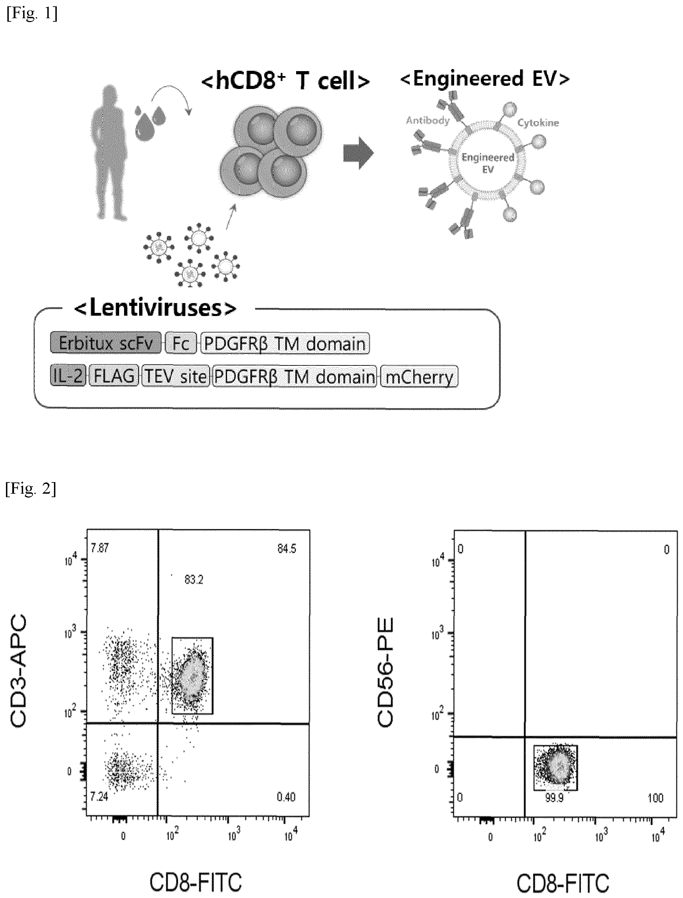

As shown in FIG. 1, in the present disclosure, immune cells expressing specific cytokines and antibodies on the surface of the cell membrane were prepared by applying the MBC platform to immune cells, thereby producing extracellular vesicles expressing specific cytokines and antibodies on the surface.

The phospholipid bilayer constituting the extracellular vesicle includes a transmembrane protein which is bound to the cytokine or antibody via a linker. Specifically, the cytokine may be IL-2 having an amino acid sequence represented by SEQ ID NO: 1, and the antibody may be scFv of Erbitux having an amino acid sequence represented by SEQ ID NO: 2, but are not limited thereto. The linker may have a 1-20 times repeating sequence of any one selected from among amino acid sequences represented by SEQ ID NOS: 3 to 13, but is not limited thereto.

The extracellular vesicle is derived from a cell transformed with a vector which includes a gene encoding a cytokine or antibody; a linker domain encoding a linker; and a transmembrane domain encoding a transmembrane protein.

Specifically, the vector for producing the extracellular vesicle includes a gene encoding interleukin-2 (IL-2) which is a cytokine or scFv of Erbitux which is an antibody, and a terminal end of the gene has a form in which the linker domain and the transmembrane domain of the platelet-derived growth factor (PDGF) receptor are sequentially linked, such that, when the vector is expressed by transforming cells therewith, the cytokine or antibody binds to the linker linked to the transmembrane protein instead of being secreted extracellularly, resulting in the cytokine or antibody expressed in a form attached to the membrane surface of the transformed cell.

The transmembrane domain may be a transmembrane domain included in any one receptor selected from the group consisting of an epidermal growth factor receptor, insulin receptor, platelet-derived growth factor (PDGF) receptor, vascular endothelial growth factor receptor, fibroblast growth factor receptor, cholecystokinin (CCK) receptor, neurotrophic factor (NGF) receptor, hepatocyte growth factor (HGF) receptor, ephrin (EPH) receptor, angiopoietin receptor, and related to receptor tyrosine kinase (RYK) receptor, but is not limited thereto.

The cell may be any one selected from the group consisting of helper T cells, CD8 + T cells, dendritic cells, neutrophil cells, eosinophil cells, basophil cells, macrophages, monocytes, natural killer cells, B cells, adult stem cells, mesenchymal stem cells, blood stem cells, and induced pluripotent stem cells (iPSc), but is not limited thereto.

The extracellular vesicle may exhibit an anticancer effect by having cancer cell-specific cytotoxicity, and it may be used as a carrier with a drug or bioactive substance encapsulated therein.

**In** addition, the present disclosure provides a pharmaceutical composition for preventing or treating cancer, comprising the extracellular vesicle as an active ingredient.

The cancer may be any one selected from the group consisting of melanoma, colon cancer, lung cancer, skin cancer, non-small cell lung cancer, bone cancer, pancreatic cancer, head or neck cancer, uterine cancer, ovarian cancer, rectal cancer, stomach cancer, anal cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic carcinoma, central nervous system tumor, primary central nervous system lymphoma, spinal cord tumor, brainstem glioma, and pituitary adenoma, but is not limited thereto.

The pharmaceutical composition may include a pharmaceutically acceptable carrier, excipient, or diluent for administration. The carrier, excipient and diluent may be selected from the group consisting of lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, and mineral oil, but is not limited thereto.

The pharmaceutical composition may be used by being formulated in the form of an oral formulation such as an acid, granule, tablet, capsule, suspension, emulsion, syrup, and aerosol as well as external agent, suppository, or sterile injection solution according to a conventional method respectively, and specifically, when formulated, it may be prepared using diluents or excipients such as a filler, extender, binder, wetting agent, disintegrant, and surfactant that are commonly used. Solid preparations for oral administration include a tablet, pill, acid, granule, capsule, and the like, but are not limited thereto. Such solid preparations may be prepared by mixing at least one or more excipients in addition to the above active ingredients, such as starch, calcium carbonate, sucrose, lactose, gelatin, and the like. **In** addition to simple excipients, lubricants such as magnesium stearate and talc may also be used. **In** addition to liquid substances and liquid paraffin for oral use, various excipients, such as a wetting agent, sweetener, aromatics, preservative, and the like, may be added for preparation. Preparations for parenteral administration include a sterilized aqueous solution, non-aqueous solvent, suspension, emulsion, lyophilized preparation, and suppository. As non-aqueous solvents and suspensions, propylene glycol, polyethylene glycol, vegetable oils such as olive oil, injectable ester such as ethyl oleate, and the like may be used. As a base of suppositories, Witepsol, Macrosol, Twin 61, cacao butter, laurin fat, glycerogelatin, and the like may be used.

Suitable dosage of the pharmaceutical composition of the present disclosure may vary depending on the condition and weight of a patient, degree of disease, drug form, and time, but may be appropriately selected by those skilled in the art, and the daily dosage of the composition may be preferably 0.001 mg/kg to 50 mg/kg and administration may be performed in single to several divided doses a day as needed.

**In** addition, the present disclosure provides a composition for delivering a drug or bioactive substance, comprising the extracellular vesicle, wherein the drug or bioactive substance is encapsulated in the extracellular vesicle.

The drug may be any one selected from the group consisting of an anticancer drug, anti-inflammatory analgesic, antibiotic, antibacterial agent and vaccine, and the bioactive substance may be any one selected from the group consisting of a peptide, protein, hormone, and gene, but are not limited thereto.

In addition, the present disclosure provides a method for producing the extracellular vesicle, including preparing a first vector comprising a gene encoding a cytokine, a linker domain encoding a linker, and a transmembrane domain encoding a transmembrane protein; preparing a second vector comprising a gene encoding an antibody, a linker domain encoding a linker, and a transmembrane domain encoding a transmembrane protein; transforming cells with the first vector and second vector using lentivirus; and isolating the extracellular vesicles from a culture medium in which the cells transformed with the first vector and second vector are cultured.

### Modes for Carrying Out the Invention

Hereinafter, to help the understanding of the present disclosure, example embodiments will be described in detail. However, the following example embodiments are merely illustrative of the contents of the present disclosure, and the scope of the present disclosure is not limited to the following example embodiments. The example embodiments of the present disclosure are provided to more completely explain the present disclosure to those of ordinary skill in the art.

### Example 1. Isolation of immune cells

Human CD8 + T lymphocytes as immune cells to be used to produce the extracellular vesicle of the present disclosure were isolated from blood. Anticoagulant-treated blood samples were treated with Ficoll, and then a buffy coat was obtained by centrifugation. The isolated buffy coat was cultured for a day in RPMI 1640 medium (Hyclone) in which 10% bovine fetal serum and 1% penicillin-streptomycin are included, and then cells suspended in the supernatant of the medium were collected to be used in the experiment. Primary CD8 + T lymphocytes were isolated from collected cells using human CD8 + T cell isolation kit (MACS) which is a kit for isolating CD8 + T lymphocytes according to the manufacturer's instructions. CD3-APC, CD8-FITC, and CD56-PE antibodies (MACS) were treated to the isolated primary CD8 + T lymphocytes, and it was found that CD8 + T lymphocytes were isolated with more than 80% accuracy via flow cytometry (FIG. 2).

### Example 2. Cell culture

HEK-293FT cells (human embryonic kidney cells) were cultured in DMEM medium (Hyclone) added with 10% bovine fetal serum and 1% penicillin-streptomycin, and A549-luc cells (human non-small cell lung cancer cells) were cultured in RPMI 1640 medium (Hyclone) added with 10% bovine fetal serum and 1% penicillin-streptomycin. Primary CD8 + T lymphocytes isolated from human blood were cultured in RPMI 1640 medium (Hyclone) added with 10% bovine fetal serum, 1% penicillin-streptomycin, 50 IU/mL of recombinant IL-2 (R&D systems), and CD3/CD28/CD2 T cell activator (STEMCELL).

### Example 3. Preparation of lentivirus

In order to produce extracellular vesicles with cytokines and antibodies expressed on the surface according to the present disclosure, prepared were a first vector comprising a gene encoding cytokines and a second vector comprising a gene encoding an antibody, each of which were transformed into cells using lentiviruses.

Specifically, the first vector includes a gene encoding IL-2 as a cytokine, a linker domain, and a transmembrane domain of a PDGF receptor, and the second vector includes a gene encoding scFv of Erbitux, a linker domain, and a transmembrane domain of the PDGF receptor. Each of the first vector and second vector was prepared with reference to the paper (Proc Natl Acad Sci U S A. 2013 May 14;110(20):8099-104).

The lentivirus to be used to transform cells with the prepared first vector and second vector was prepared in the following manner. HEK-293FT cells were inoculated into a 100 pi plate containing DMEM (Hyclone) medium at a cell concentration of about 5 X 10⁶. pCMVD (# PS100001; Origene) and pVSVg (# 1733; Addgene) viral vectors were mixed with the first vector or second vector in a ratio of 1:1:1, and the mixture was treated to cells using Turbofect (Thermo fisher scientific) according to the manufacturer's instructions, followed by overnight culture at 37°C. After culture, the supernatant was removed, and the medium was replaced with fresh DMEM medium including 10% bovine fetal serum and 1% penicillin-streptomycin. After 48 hours of culture, the virus-included supernatant of medium was obtained and centrifuged, and a surfactant-free cellulose acetate (SFCA) membrane filtration device (0.45 µm; Corning) was used to remove cell debris. The titer of each lentivirus was measured by the Lenti-X p24 rapid titer kit (# 632200; Takara) according to the manufacturer's instructions. First lentivirus including the first vector and second lentivirus including the second vector were each dispensed and frozen at -80°C to be stored.

### Example 4. Transformation of immune cells using lentivirus

In order to produce the extracellular vesicles that express cytokines and antibodies on the surface according to the present disclosure, immune cells were transformed with lentiviruses prepared in Example 3.

To prepare transformed immune cells, the first lentivirus or the second lentivirus prepared in Example 3 was treated to primary CD8 + T lymphocytes along with 10 µg/mL of polybrene at a concentration of 100 multiplicity of infection (MOI). The cell mixture treated with each lentivirus was centrifuged at 1,200 g at 30°C for 90 minutes and then subjected to spinoculation, followed by overnight culture at 37°C. The surplus virus was removed, followed by medium replacement with 10% bovine fetal serum-added fresh DMEM medium including 10% bovine fetal serum.

Untransformed lymphocyte C was used as a control, and marking was followed as lymphocyte I transformed only with first lentivirus including the IL-2 gene, lymphocyte E transformed only with second lentivirus including the scFv gene of Erbitux, and lymphocyte D transformed with both the lentiviruses.

### Example 5. Identification of expression of cytokines and antibodies in transformed immune cells

In order to produce extracellular vesicles that express cytokines and antibodies on the surface according to the present disclosure, identified was whether cytokines and antibodies are expressed in the transformed immune cells prepared in Example 4.

In Example 4, mRNA was extracted from lymphocytes transformed with the first lentivirus and/or second lentivirus using a kit (mRNA extraction kit, # 9767; TaKaRa) according to the manufacturer's instructions, and the concentration of the extracted mRNA was measured using nanodrop (DS-11 Series Spectrophotometer; DeNovix). 100 ng of extracted mRNA was synthesized into cDNA using a kit (SuperScript III First-Strand Synthesis System, Ref. 18080-051; invitrogen) according to the manufacturer's instructions.

The expression level of the IL-2 gene and/or the scFv gene of Erbitux in each lymphocyte was analyzed by real-time PCR using a primer set shown in Table 1 below.

**TABLE 1**

| Gene | | Sequences | SEQ ID NO. |
|---|---|---|---|
| IL-2 | F | AGACCCAGGGACTTAATCAG | 14 |
| | R | ACAATGGTTGCTGTCTCATC | 15 |
| Erbitux | F | GGCCCAGCCGGCCAT | 16 |
| | R | ATTTAGGCCCCCGAGGCCTTTCAGCTCCAG CTTGGTCCCA | 17 |

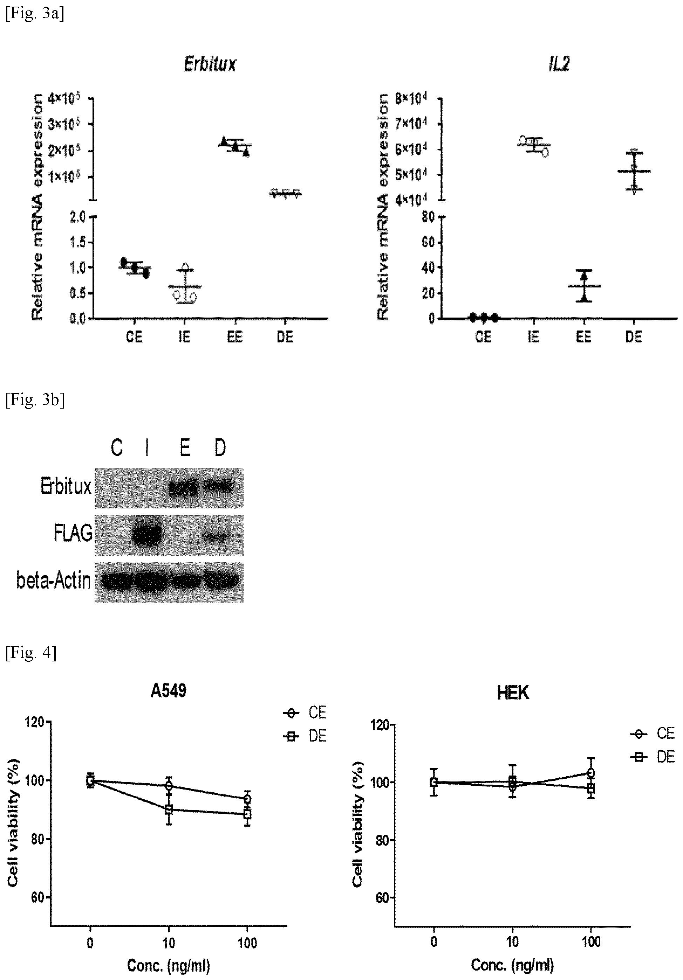

As shown in FIG. 3, respective expression of the IL-2 gene in the transformed lymphocytes compared to the control increased about 10⁴ times, and the expression level of the scFv gene of Erbitux increased about 10⁵ times. In particular, D lymphocytes transformed with both lentiviruses showed an increase in the expression level of both the IL-2 gene and/or the scFv gene of Erbitux.

In addition, the expression level of each protein was measured by Western blot to identify the expression level of IL-2 and/or Erbitux in the primary CD8 + T lymphocytes. Cells were separated by SDS-PAGE and transferred to a nitrocellulose membrane. Then, a reaction was carried out with the primary antibody (R&D system, #MAB9626) of Erbitux, the primary antibody (CST, # 8146) of FLAG, and beta-actin (CST, # 4970), followed by a reaction with HRP-conjugated secondary antibodies. Enhanced chemiluminescence (ECL) detection reagent (# 34095; Thermo Scientific, # RPN2209; GE Healthcare) was used to visualize the image.

As shown in FIG. 3, only IL-2 was expressed in I lymphocytes transformed only by the first lentivirus while only Erbitux was expressed in E lymphocytes transformed only by the second lentivirus, wherein both IL-2 and Erbitux were expressed in D lymphocytes transformed by both lentiviruses.

### Example 6. Isolation and purification of extracellular vesicles

In order to produce the extracellular vesicle that express cytokines and antibodies on the surface according to the present disclosure, the extracellular vesicles were isolated from the transformed immune cells which were prepared in Example 4.

Control C lymphocytes or transformed D lymphocytes in Example 4 were inoculated in bovine fetal serum-free RPMI medium at a concentration of 1 x 10⁶ cells, respectively, followed by culture for 72 hours. The supernatant of the culture medium in which each cell was cultured was sequentially subjected to continuous centrifugation at 300 g, 2,500 g, and 10,000 g. The centrifuged supernatant was filtered through a 0.2 µm syringe filter and centrifuged at 120,000 g to obtain pellets of the extracellular vesicles. The obtained pellets were suspended in PBS and cryopreserved at -80°C.

The extracellular vesicles obtained from untransformed lymphocytes C were labeled as CE, and the extracellular vesicles obtained from lymphocyte D transformed with first and second lentiviruses as DE.

### Example 7. Analysis of cancer cell-specific cytotoxicity by extracellular vesicles

In order to evaluate the cancer cell-specific cytotoxicity of the extracellular vesicle produced according to the present disclosure, the cytotoxicity of the extracellular vesicle for cancer cells or normal cells was analyzed.

A549-luc cells which are human non-small cell lung cancer cells and HEK cells which are normal cells were cultured in 96 well plates at a concentration of 5 x 10³ cells/well, respectively, for a day. The extracellular vesicles produced in Example 6 were treated to cells at concentrations of 0 ng/ml, 10 ng/ml, or 100 ng/ml, respectively, followed by culture for 48 hours. The degree of cell death was analyzed with Cell Titer 96 AQueous Assay (Promega, #G5421).

As shown in FIG. 4, the cell death did not take place in HEK cells which are normal cells even upon treatment of the extracellular vesicles, but in the A549-luc cells which are cancer cells upon treatment of the extracellular vesicles. In particular, when treated with extracellular vesicle DE obtained from the lymphocytes D transformed by the first and second lentiviruses, the degree of cancer cell death was greater, compared to the case treated with extracellular vesicle CE obtained from untransformed lymphocytes C.

The above results demonstrate that, since the extracellular vesicles of the present disclosure exhibited cancer cell-specific cytotoxicity to kill only cancer cells, it may be used as a safe anticancer agent capable of eliminating only cancer cells without affecting normal cells.

### Example 8. Analysis of IL-2-induced cytotoxicity

In order to determine whether the cytotoxicity shown in Example 7 was induced by IL-2, cytotoxicity was evaluated in the same manner as in Example 7, except for treatment of IL-2 instead of the extracellular vesicles.

A549-luc cells which are human non-small cell lung cancer cells as well as HEK cells which are normal cells were cultured in 96 well plates at a concentration of 5 x 10³ cells/well, respectively, for a day. The IL-2 prepared in Example 6 was treated to cells at concentrations of 0 IU/ml, 10 IU/ml, or 100 IU/ml, respectively, followed by culture for 48 hours. The degree of cell death was analyzed by Cell Titer 96 AQueous Assay (Promega, #G5421).

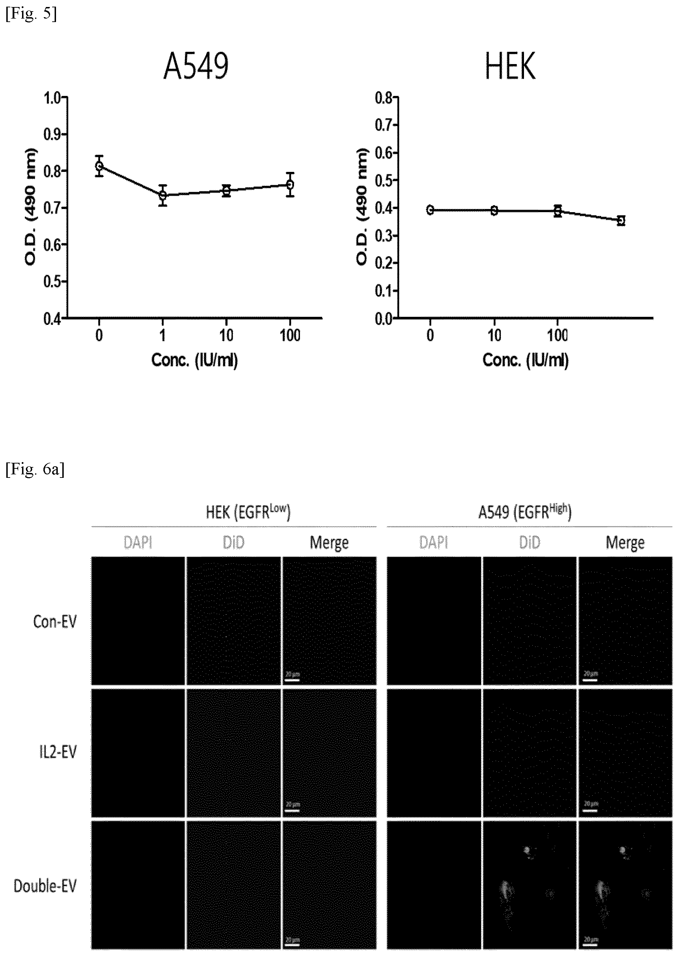

As shown in FIG. 5, IL-2 did not induce cytotoxicity in both cancer cells or normal cells, and the cytotoxicity shown in Example 7 was not induced by IL-2 that was expressed on the surface of the extracellular vesicles, but the cancer cell-specific cytotoxicity was shown by the extracellular vesicle itself.

### Example 9. Evaluation of cancer cell specificity of extracellular vesicles with antibodies expressed on the surface

In order to evaluate the cancer cell specificity of the extracellular vesicles with the antibodies expressed on the surface according to the present disclosure, the extracellular vesicles in Example 6 were labeled with fluorescence to analyze the specificity of cancer cells.

A549-luc cells which are human non-small cell lung cancer cells as well as HEK cells which are normal cells were cultured in an 8 well chamber at a concentration of 1 x 10⁴ cells/wells for a day. Extracellular vesicles stained with DID (Molecular Probes, #V-22887) was treated to cultured cells at a concentration of 100 ng/ml for 3 hours. Thereafter, the cells were washed once with phosphate buffer saline (PBS), fixed with 4% formaldehyde, washed three times with saline, and treated with DAPI to stain the nucleus. Images of the stained cells were analyzed using a confocal microscope.

In addition, A549-luc cells which are human non-small cell lung cancer cells as well as HEK cells which are normal cells were cultured in 12 well plates at a concentration of 1 x 10⁴ cells/well, the concentration of the extracellular vesicle stained with DID (Molecular Probes, #V-22887) was adjusted to 100 ng/ml, and washing and fixing were performed in the same process as above, followed by analysis with a flow cytometer.

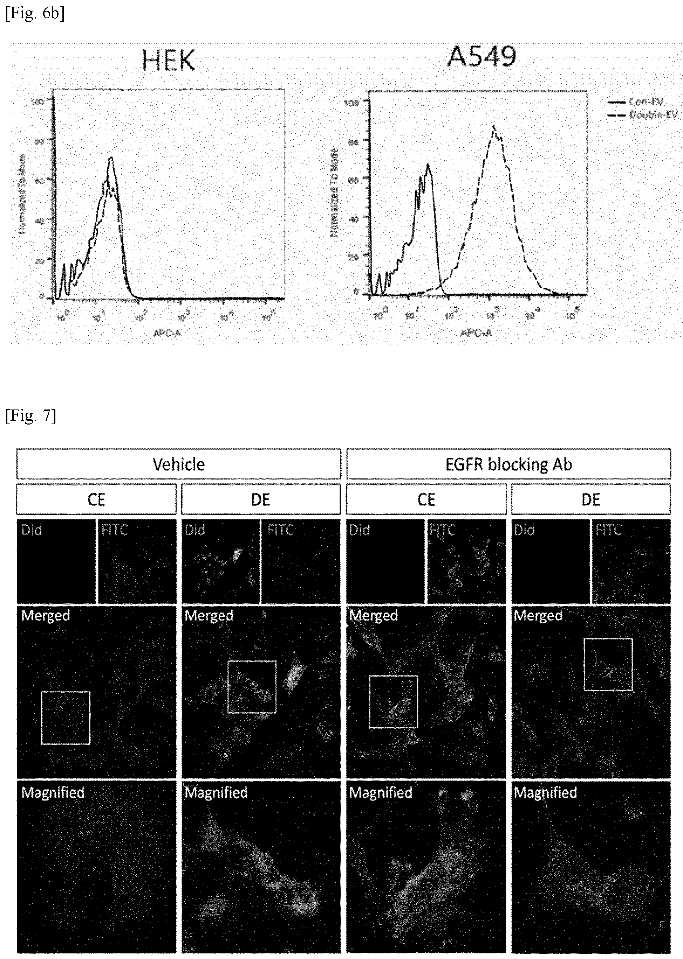

As shown in FIG. 6, when treated with extracellular vesicles (Con-EV) obtained from untransformed lymphocytes C and extracellular vesicles (IL2-EV) obtained from lymphocyte I transformed only with the first lentivirus, no observation was made due to the extracellular vesicles failed to bind to normal cells or cancer cells, but, when treated with extracellular vesicles (Double-EV) obtained from lymphocyte D transformed with the first and second lentiviruses, the binding of the DID-stained extracellular vesicle only to cancer cells was observed.

This demonstrates that only the extracellular vesicle (Double-EV) expressing antibodies on the surface bound to the cancer cells by recognizing the same, thereby having cancer cell specificity.

### Example 10. Verification of cancer cell specificity of extracellular vesicles with antibodies expressed on the surface

In order to verify the cancer cell specificity of the extracellular vesicle with antibodies expressed on the surface according to the present disclosure, competitive antibodies were treated prior to treatment of the extracellular vesicle, followed by analysis on whether the cancer cell specificity of the extracellular vesicle was retained.

The extracellular vesicles prepared in Example 6 expressed scFv of Erbitux as an antibody, and the scFv of Erbitux bound to a epidermal growth factor receptor (EGFR) which is overexpressed in cancer cells. Thus, antibodies that competitively bind to EGFR were used to verify cancer cell specificity due to the antibody in the extracellular vesicles.

A549-luc cells which are human non-small cell lung cancer cells as well as HEK cells which are normal cells were cultured in an 8 well chamber at a concentration of 1 x 10⁴ cells/well. Each cell was pretreated with competitive antibodies that specifically bind to EGFR for 1 hour, and then extracellular vesicles stained with DID (Molecular Probes, #V-22887) were treated at a concentration of 100 ng/ml for 3 hours. Cells were washed once with phosphate buffer saline (PBS) and fixed with 4% formaldehyde. After washing with saline three times, a reaction with human Fc-recognizing secondary antibody (with FITC fluorescent attached) was performed at room temperature for 1 hour, followed by washing with saline three times. After washing three times with saline, DAPI was treated to stain the nucleus. Images of the stained cells were analyzed using a confocal microscope.

As shown in FIG. 7, it was determined that the extracellular vesicle expressing antibodies maintained cancer cell specificity as in Example 9, but no extracellular vesicle was observed in cancer cells pretreated with competitive antibodies that specifically bind to EGFR. This means that when the EGFR of cancer cells are masked by competitive antibodies, the extracellular vesicles do not recognize cancer cells. Thus, the result demonstrates that the extracellular vesicles of the present disclosure target cancer cells by antibodies expressed on the surface.

### Example 11. Verification of antibody-specific targeting of extracellular vesicles in animal models

To verify the likelihood of cancer cell-specific targeting by antibodies attached to the surface, fluorescence-stained extracellular vesicles were treated to a non-small cell lung cancer cell line (A549)-grafted mouse model.

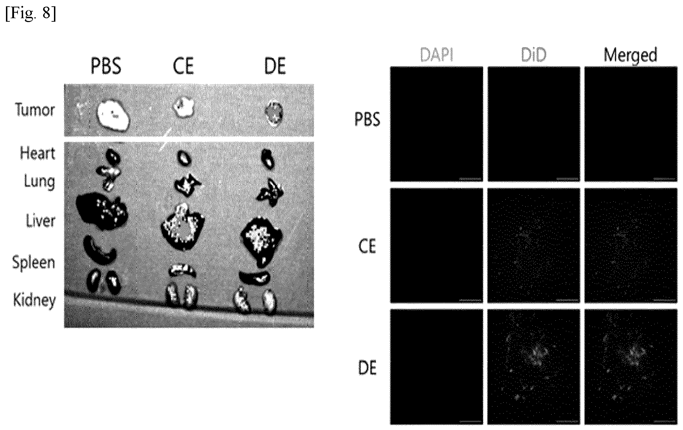

After purchasing BALB/c nude mice (male, 6 weeks old) and undergoing 1 week of stabilization, the non-small cell lung cancer cell line (A549) was subjected to subcutaneous injection in the count of 5 x 10⁶ cells/mice and left until the tumor volume becomes 100 mm³. The tumor volume was calculated by long axis x (short axis)² x 0.5. After the tumor volume became 100 mm³, 20 µg of extracellular vesicles stained by DID (Molecular Probes, #V-22887) was injected via mouse tail vein injection. 24 hours after the injection of extracellular vesicles, mice were sacrificed by cervical dislocation, and then each organ (tumor, heart, lung, liver, spleen, and kidney) was subjected to dissection to detect fluorescence intensity using a 'fluorescence, luminescence, and computed tomography in vivo optical imaging system (IVIS)'. As a result, as shown on the left image in FIG. 8, it was found that the greater number of extracellular vesicle (DE) expressing antibodies on the surface was delivered to the tumor to emit strong fluorescence. (PBS; Phosphate-buffered saline injected control, CE; Control extracellular vesicle treated group, DE; Extracellular vesicle treated group expressing both IL-2 and Erbitux)

In addition, to prepare a tumor cryosection, the tumor was fixed with 4% formaldehyde and washed three times with PBS, followed by overnight incubation in 30% sucrose solution. The tumor was then fixed at -20°C with OCT compound and placed on a slide after cryosection, followed by analysis of fluorescence intensity through confocal fluorescence microscopy after nuclear staining with DAPI. As shown on the right image in FIG. 8, it was found that the extracellular vesicle (DE) expressing antibodies on its surface was observed more in the cross-section of the tumor cryosection. (PBS; Phosphate-buffered saline injected control, CE; Control extracellular vesicle treated group, DE; Extracellular vesicle treated group expressing both IL-2 and Erbitux)

## Claims

1. An extracellular vesicle expressing cytokines and antibodies on its surface, wherein the cytokine is IL-2 having an amino acid sequence represented by SEQ ID NO: 1, and the extracellular vesicle is derived from T cells.

2. The extracellular vesicle of claim 1, wherein a phospholipid bilayer constituting the extracellular vesicle comprises a transmembrane protein which is bound to the cytokine or antibody via a linker.

3. The extracellular vesicle of claim 2, wherein the antibody is scFv of Erbitux having an amino acid sequence represented by SEQ ID NO: 2.

4. The extracellular vesicle of claim 2, wherein the linker has a 1-20 times repeating sequence of any one selected from among amino acid sequences represented by SEQ ID NOS: 3 to 13.

5. The extracellular vesicle of claim 2, wherein the extracellular vesicle is derived from T cells transformed with a vector which comprises a gene encoding a cytokine or antibody; a linker domain encoding a linker; and a transmembrane domain encoding a transmembrane protein.

6. The extracellular vesicle of claim 5, wherein the transmembrane domain is a transmembrane domain included in any one receptor selected from the group consisting of an epidermal growth factor receptor, insulin receptor, platelet-derived growth factor (PDGF) receptor, vascular endothelial growth factor receptor, fibroblast growth factor receptor, cholecystokinin (CCK) receptor, neurotrophic factor (NGF) receptor, hepatocyte growth factor (HGF) receptor, ephrin (EPH) receptor, angiopoietin receptor, and related to receptor tyrosine kinase (RYK) receptor.

7. The extracellular vesicle of claim 5, wherein the T cell is a helper T cell or a CD8 + T cell.

8. The extracellular vesicle of claim 1, wherein the extracellular vesicle exhibits an anticancer effect by having cancer cell-specific cytotoxicity.

9. The extracellular vesicle of claim 8, wherein the extracellular vesicle is used as a carrier with a drug or bioactive substance encapsulated therein.

10. A pharmaceutical composition for use in preventing or treating cancer, comprising the extracellular vesicle of any one of claims 1 to 9 as an active ingredient.

11. The pharmaceutical composition for use of claim 10, wherein the cancer is any one selected from the group consisting of melanoma, colon cancer, lung cancer, skin cancer, non-small cell lung cancer, bone cancer, pancreatic cancer, head or neck cancer, uterine cancer, ovarian cancer, rectal cancer, stomach cancer, anal cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic carcinoma, central nervous system tumor, primary central nervous system lymphoma, spinal cord tumor, brainstem glioma, and pituitary adenoma.

12. A composition for use in delivering a drug or bioactive substance, comprising the extracellular vesicle of any one of claims 1 to 9, wherein the drug or bioactive substance is encapsulated in the extracellular vesicle.

13. The composition for use of claim 12, wherein the drug is any one selected from the group consisting of an anticancer drug, anti-inflammatory analgesic, antibiotic, antibacterial agent and vaccine, and the bioactive substance is any one selected from the group consisting of a peptide, protein, hormone, and gene.

14. A method for producing the extracellular vesicle of any one of claims 1 to 9, comprising:
preparing a first vector comprising a gene encoding a cytokine, a linker domain encoding a linker, and a transmembrane domain encoding a transmembrane protein;
preparing a second vector comprising a gene encoding an antibody, a linker domain encoding a linker, and a transmembrane domain encoding a transmembrane protein;
transforming cells with the first vector and second vector using lentivirus; and
isolating the extracellular vesicle from a culture medium in which the cells transformed with the first vector and second vector are cultured.

## Patentansprüche

1. Extrazelluläres Vesikel, das Zytokine und Antikörper an seiner Oberfläche exprimiert, wobei das Zytokin IL-2 mit einer Aminosäuresequenz ist, die durch SEQ ID NO: 1 dargestellt wird, und das extrazelluläre Vesikel aus T-Zellen stammt.

2. Extrazelluläres Vesikel nach Anspruch 1, wobei eine Phospholipid-Doppelschicht, die das extrazelluläre Vesikel bildet, ein Transmembranprotein umfasst, das über einen Linker an das Zytokin oder den Antikörper gebunden ist.

3. Extrazelluläres Vesikel nach Anspruch 2, wobei der Antikörper scFv von Erbitux mit einer Aminosäuresequenz ist, die durch SEQ ID NO: 2 dargestellt wird.

4. Extrazelluläres Vesikel nach Anspruch 2, wobei der Linker eine 1- bis 20-mal wiederholte Sequenz aufweist, die aus einer der durch SEQ ID NOS: 3 bis 13 dargestellten Aminosäuresequenzen ausgewählt ist.

5. Extrazelluläres Vesikel nach Anspruch 2, wobei das extrazelluläre Vesikel aus T-Zellen stammt, die mit einem Vektor transformiert wurden, der ein Gen, das ein Zytokin oder einen Antikörper kodiert, eine Linker-Domäne, die einen Linker kodiert, und eine Transmembrandomäne, die ein Transmembranprotein kodiert, umfasst.

6. Extrazelluläres Vesikel nach Anspruch 5, wobei die Transmembrandomäne eine Transmembrandomäne ist, die in einem Rezeptor enthalten ist, der aus der Gruppe bestehend aus einem epidermalen Wachstumsfaktorrezeptor, einem Insulinrezeptor, einem Plättchen-Wachstumsfaktor (PDGF)-Rezeptor, einem vaskulären endothelialen Wachstumsfaktorrezeptor, einem Fibroblasten-Wachstumsfaktor-Rezeptor, einem Cholecystokinin (CCK)-Rezeptor, einem neurotropher Faktor (NGF)-Rezeptor, einem Hepatozyten-Wachstumsfaktor (HGF)-Rezeptor, einem Ephrin (EPH)-Rezeptor, einem Angiopoietin-Rezeptor und einem Rezeptor-Tyrosinkinase (RYK)-Rezeptor ausgewählt ist.

7. Extrazelluläres Vesikel nach Anspruch 5, wobei die T-Zelle eine Helfer-T-Zelle oder eine CD8+-T-Zelle ist.

8. Extrazelluläres Vesikel nach Anspruch 1, wobei das extrazelluläre Vesikel eine krebsbekämpfende Wirkung aufweist, indem es eine Krebszell-spezifische Zytotoxizität besitzt.

9. Extrazelluläres Vesikel nach Anspruch 8, wobei das extrazelluläre Vesikel als Träger mit einem darin eingekapselten Arzneimittel oder einer bioaktiven Substanz verwendet wird.

10. Pharmazeutische Zusammensetzung zur Verwendung bei der Vorbeugung oder Behandlung von Krebs, umfassend das extrazelluläre Vesikel nach einem der Ansprüche 1 bis 9 als Wirkstoff.

11. Pharmazeutische Zusammensetzung zur Verwendung nach Anspruch 10, wobei der Krebs ausgewählt ist aus der Gruppe bestehend aus Melanom, Darmkrebs, Lungenkrebs, Hautkrebs, nicht-kleinzelliger Lungenkrebs, Knochenkrebs, Bauchspeicheldrüsenkrebs, Kopf-oder Halskrebs, Gebärmutterkrebs, Eierstockkrebs, Rektumkrebs, Magenkrebs, Analkrebs, Brustkrebs, Eileiterkrebs, Gebärmutterkrebs, Gebärmutterhalskrebs, Vaginalkrebs, Vulvakrebs, Hodgkin-Krankheit, Speiseröhrenkrebs, Dünndarmkrebs, Krebs der endokrinen Drüsen, Schilddrüsenkrebs, Nebenschilddrüsenkrebs, Nebennierenkrebs, Weichteilsarkom, Harnröhrenkrebs, Peniskrebs, Prostatakrebs, chronische oder akute Leukämie, lymphozytisches Lymphom, Blasenkrebs, Nieren- oder Harnleiterkrebs, Nierenzellkarzinom, Nierenbeckenkarzinom, Tumor des Zentralnervensystems, primäres Lymphom des Zentralnervensystems, Tumor des Rückenmarks, Hirnstammgliom und Hypophysenadenom.

12. Zusammensetzung zur Verwendung bei der Verabreichung eines Arzneimittels oder einer bioaktiven Substanz, umfassend das extrazelluläre Vesikel nach einem der Ansprüche 1 bis 9, wobei das Arzneimittel oder die bioaktive Substanz in dem extrazellulären Vesikel eingekapselt ist.

13. Zusammensetzung zur Verwendung nach Anspruch 12, wobei das Arzneimittel ausgewählt ist aus der Gruppe bestehend aus einem Krebsmedikament, einem entzündungshemmenden Analgetikum, einem Antibiotikum, einem antibakteriellen Mittel und einem Impfstoff ausgewählt ist, und die bioaktive Substanz aus der Gruppe bestehend aus einem Peptid, einem Protein, einem Hormon und einem Gen.

14. Verfahren zur Herstellung des extrazellulären Vesikels nach einem der Ansprüche 1 bis 9, umfassend:
Herstellen eines ersten Vektors umfassend ein Gen, das ein Zytokin kodiert, eine Linker-Domäne, die einen Linker kodiert, und eine Transmembrandomäne, die ein Transmembranprotein kodiert;
Herstellen eines zweiten Vektors umfassend ein Gen, das einen Antikörper kodiert, eine Linker-Domäne, die einen Linker kodiert, und eine Transmembrandomäne, die ein Transmembranprotein kodiert;
Transformieren von Zellen mit dem ersten Vektor und dem zweiten Vektor unter Verwendung eines Lentivirus; und
Isolieren der extrazellulären Vesikel aus einem Kulturmedium, in dem die mit dem ersten Vektor und dem zweiten Vektor transformierten Zellen kultiviert werden.

## Revendications

1. Vésicule extracellulaire exprimant des cytokines et des anticorps à sa surface, dans laquelle la cytokine est l'IL-2 possédant une séquence d'acides aminés représentée par SEQ ID NO : 1, et la vésicule extracellulaire est dérivée de cellules T.

2. Vésicule extracellulaire selon la revendication 1, dans laquelle une bicouche phospholipidique constituant la vésicule extracellulaire comprend une protéine transmembranaire qui est liée à la cytokine ou à l'anticorps via un lieur.

3. Vésicule extracellulaire selon la revendication 2, dans laquelle l'anticorps est un scFv d'Erbitux possédant une séquence d'acides aminés représentée par SEQ ID NO : 2.

4. Vésicule extracellulaire selon la revendication 2, dans laquelle le lieur comporte une séquence répétée 1 à 20 fois choisie parmi les séquences d'acides aminés représentées par les SEQ ID NOS : 3 à 13.

5. Vésicule extracellulaire selon la revendication 2, dans laquelle la vésicule extracellulaire est dérivée de cellules T transformées avec un vecteur qui comprend un gène codant une cytokine ou un anticorps ; un domaine de liaison codant un lieur ; et un domaine transmembranaire codant une protéine transmembranaire.

6. Vésicule extracellulaire selon la revendication 5, dans laquelle le domaine transmembranaire est un domaine transmembranaire inclus dans l'un quelconque des récepteurs choisis parmi le groupe constitué par un récepteur du facteur de croissance épidermique, un récepteur de l'insuline, un récepteur du facteur de croissance dérivé des plaquettes (PDGF), un récepteur du facteur de croissance endothélial vasculaire, un récepteur du facteur de croissance des fibroblastes, un récepteur de la cholécystokinine (CCK), un récepteur du facteur de croissance nerveux (NGF), un récepteur du facteur de croissance hépatocytaire (HGF), un récepteur de l'éphrine (EPH), un récepteur de l'angiopoïétine et un récepteur apparenté au récepteur tyrosine kinase (RYK).

7. Vésicule extracellulaire selon la revendication 5, dans laquelle la cellule T est une cellule T auxiliaire ou une cellule T CD8+.

8. Vésicule extracellulaire selon la revendication 1, dans laquelle la vésicule extracellulaire présente un effet anticancéreux en ayant une cytotoxicité spécifique aux cellules cancéreuses.

9. Vésicule extracellulaire selon la revendication 8, dans laquelle la vésicule extracellulaire est utilisée comme support avec un médicament ou une substance bioactive encapsulée à l'intérieur.

10. Composition pharmaceutique pour utilisation dans la prévention ou le traitement du cancer, comprenant la vésicule extracellulaire selon l'une quelconque des revendications 1 à 9 en tant qu'ingrédient actif.

11. Composition pharmaceutique pour utilisation selon la revendication 10, dans laquelle le cancer est choisi parmi le groupe comprenant le mélanome, le cancer du côlon, le cancer du poumon, le cancer de la peau, le cancer du poumon non à petites cellules, le cancer des os, le cancer du pancréas, le cancer de la tête ou du cou, le cancer de l'utérus, le cancer de l'ovaire, le cancer du rectum, le cancer de l'estomac, le cancer de l'anus, le cancer du sein, le carcinome des trompes de Fallope, le carcinome de l'endomètre, le carcinome du col de l'utérus, le carcinome vaginal, le carcinome vulvaire, la maladie de Hodgkin, le cancer de l'œsophage, le cancer de l'intestin grêle, le cancer des glandes endocrines, le cancer de la thyroïde, le cancer des parathyroïdes, le cancer des surrénales, le sarcome des tissus mous, le cancer de l'urètre, le cancer du pénis, le cancer de la prostate, la leucémie chronique ou aiguë, le lymphome lymphocytaire, le cancer de la vessie, le cancer du rein ou de l'uretère, le carcinome rénal, le carcinome du bassinet rénal, la tumeur du système nerveux central, le lymphome primaire du système nerveux central, tumeur de la moelle épinière, gliome du tronc cérébral et adénome hypophysaire.

12. Composition pour utilisation dans l'administration d'un médicament ou d'une substance bioactive, comprenant la vésicule extracellulaire de l'une quelconque des revendications 1 à 9, dans laquelle le médicament ou la substance bioactive est encapsulé dans la vésicule extracellulaire.

13. Composition pour utilisation selon la revendication 12, dans laquelle le médicament est choisi parmi le groupe comprenant un médicament anticancéreux, un analgésique anti-inflammatoire, un antibiotique, un agent antibactérien et un vaccin, et la substance bioactive est choisie parmi le groupe comprenant un peptide, une protéine, une hormone et un gène.

14. Procédé de production de la vésicule extracellulaire selon l'une quelconque des revendications 1 à 9, comprenant :
préparer un premier vecteur comprenant un gène codant une cytokine, un domaine de liaison codant un lieur et un domaine transmembranaire codant une protéine transmembranaire ;
préparer un deuxième vecteur comprenant un gène codant un anticorps, un domaine de liaison codant un lieur et un domaine transmembranaire codant une protéine transmembranaire ;
transformer des cellules avec le premier vecteur et le deuxième vecteur en utilisant un lentivirus ; et
isoler la vésicule extracellulaire à partir d'un milieu de culture dans lequel les cellules transformées avec le premier vecteur et le deuxième vecteur sont cultivées.
